# EUROPEAN PATENT SPECIFICATION

(11) **EP 1 958 626 B1**
(45) Date of publication and mention of the grant of the patent: **23.07.2014**
(21) Application number: 08250527.2
(22) Date of filing: 13.02.2008
(51) Int. Cl.: A61K 31/198, C12N 7/06, A23L 1/305

(54) **Method for inactivating viruses by addition of slightly acidic arginine**
Verfahren zur Inaktivierung von Viren durch Zusatz von leicht saurem Arginin
Procédé pour l'inactivation de virus par ajout d'une arginine légèrement acide

(30) Priority: 13.02.2007 US 889554 P; 03.12.2007 US 991831 P
(43) Date of publication of application: 20.08.2008
(73) Proprietor: Ajinomoto Co., Inc., Tokyo 104-8315 (JP)
(72) Inventor: Koyama, Hajime, Wakayama 641-8509 (JP); Arakawa, Tsutomu, Thousand Oaks, CA 93160 (US); Ejima, Daisuke, Kanagawa 210-8681 (JP)
(74) Representative: Nicholls, Kathryn Margaret

(56) References cited:
- WO-A-2007/016450
- US-A- 5 110 600
- US-A1- 2003 087 827
- DOCHERTY J J ET AL: "Inactivation of Herpes simplex virus types 1 and 2 by synthetic histidine peptides" ANTIMICROBIAL AGENTS AND CHEMOTHERAPY, vol. 31, no. 10, October 1987 (1987-10), pages 1562-1566, XP002480022
- KOZLOFF L M ET AL: "Critical arginine residue for maintaining the bacteriophage tail structure" JOURNAL OF VIROLOGY, vol. 3, no. 2, 1969, pages 217-227, XP002480023
- CABRERA C ET AL: "Anti-human immunodeficiency virus activity of novel aminoglycoside-arginine conjugates at early stages of infection" AIDS RESEARCH AND HUMAN RETROVIRUSES, MARY ANN LIEBERT, US, vol. 16, no. 7, 1 May 2000 (2000-05-01), pages 627-634, XP002370082 ISSN: 0889-2229

## Description

The present invention relates to a virus inactivation method, which is necessary for the preparation of protein formulations, in the presence of arginine under more moderate pH conditions than those in the conventional acid pH treatment for virus inactivation.

In the manufacture of protein formulations, it is absolutely required to provide a step of highly inactivating or eliminating viruses with which the protein formulations may be contaminated during the manufacturing process (ICH Harmonized Tripartite Guideline: Viral Safety Evaluation of Biotechnology Products Derived from Cell lines of Human or Animal Origin).

To inactivate the viruses, a plurality of treatments based on different mechanisms are conventionally employed, for example, the pasteurization where heat treatment of about 60°C is continuously conducted for about ten hours; the solvent/detergent (S/D) treatment where the protein formulations are exposed to a solution designed for virus inactivation comprising an organic solvent such as tris-(n-butyl)-phosphate (TNBP) or the like and a surfactant such as Tween-80 or the like; the chemical treatment where the protein formulations are exposed to particular chemical substances, such as an organic acid, e.g., caprylic acid or the like, an alcohol having 4 to 10 carbon atoms, β-propiolactone, and the like; the photochemical inactivation treatment where a photosensitive compound such as psoralen or the like is used in combination with ultraviolet irradiation; the treatment by gamma irradiation, and so on (Sofer, et al. BioPharm International, October 42-51, 2002).

However, the target proteins are at risk of being denatured or decomposed under the severe environmental conditions created by any of the above-mentioned inactivation methods. In the case where any inactivating agent is added to the protein, it is necessary to separate and remove the inactivating agent from the protein formulation at any stage after the inactivation step.

Certain viruses coated with lipid envelope are known to drastically lose their infectivity just by being exposed under acidic pH conditions at low temperatures for a short period of time (0.5 to one hour). In light of this, the virus inactivation method based on the acid treatment using an acid such as citric acid or the like has been introduced in the production process of various kinds of protein formulations (Brorson, et al. Biotechnology and Bioengineering 82, 321-329, 2003). The above-mentioned method is a remarkably simple process for virus inactivation. To be more specific, the protein solution is adjusted to pH 5 or less using a buffer solution serving as a pH adjustor and the protein formulation is then retained at a selected temperature ranging from about 0 to about 30°C for a short period of time, whereby the inactivation reaction proceeds. Once the formulation is neutralized using a base, the production process of the formulation can be restarted. In this case, virus inactivation is triggered merely by the acid pH treatment, so that any particular chemical substances are not needed, and accordingly, no extra step of removing such chemical substances will be required. Although the acid sensitivity varies among the viruses, the previous reports have revealed that the exposure to more acidic conditions than pH 3.5-4 is required to effectively inactivate the viruses (Sofer, et.al. BioPharm International, April 42-68, 2003; Burstyn, et al. Developments in Biological Standardization 88, 73-79 (1996)). For example, Louie et al. made a study of how to inactivate the bovine viral diarrhea virus (BVDV) by acid treatment, and consequently found that the BVDV cannot be completely inactivated even when subjected to the treatment at pH 4.25 and 21°C for 21 days (Louie, et al. Biologicals 22, 13-19, 1994). Some protein formulations use tough proteins that do not cause denaturation or decomposition even when exposed to strongly acidic pH conditions; others use proteins such as antibodies which are prone to denaturation or association under the strongly acidic conditions (Paborji, M. et al: Pharmaceutical Research. 11, 764-771 (1994)). From the viewpoint of quality assurance of target proteins, due consideration is required to apply the acid treatment to the proteins that may cause denaturation or association under the strongly acidic conditions. Thus, there has been a demand for a virus inactivation method based on moderate acid treatment, i.e., under slightly acidic pH conditions, capable of producing the virus inactivating effect similarly to the methods based on the acid treatment using strong acids.

Milton et al. found a method based on combination of the S/D treatment and the acid treatment for inactivating the viruses in the preparation of immunoglobulin formulations (EP0523406). The above-mentioned combination method where the S/D treatment is conducted at pH 4 to 4.85 largely improves the inactivation efficiency when compared with the conventional S/D methods. Although the combination method can make the pH conditions more moderate, it becomes necessary to remove the organic solvent and the surfactant added to the protein formulation. Juergen et al. found a method for preparing immunoglobulin formulations substantially free from viruses, which method employs the step of inactivating the viruses by exposing the protein formulation to caprylic acid or heptanoic acid at pH 4.6 to 4.95 (WO2005082937). Johnston et al. demonstrated that the titer of BVDV was decreased to 1/10000 when the formulation was exposed to 16 mM caprylic acid at 30°C for 10 hours under the conditions of pH 4.5, and then proposed an inactivation method using an acid treatment and a chemical substance in combination (Biologicals 31, 213-221 (2003)). However, those preparation methods need the subsequent step of removing caprylic acid or the like added for virus inactivation, although the pH conditions surely become moderate. In addition, it has been known for 40 years and more that the viruses contained in protein can be effectively inactivated when a slight amount of pepsin is allowed to exist together with the protein and the obtained system is adjusted to pH 4.0 (Jensch, et al. Transfusion 31, 423-427 (1991); Kempf, et al. Transfusion 36, 866-872 (1996)). Although the pH conditions are made moderate, the intentional addition of pepsin, i.e., one of the proteases, to the target protein is not considered to be an advantageous choice in ensuring the quality of the target protein.

Arginine, one of the amino acids is known to inhibit the nonspecific association and aggregation reactions between the proteins and also known as a chemical to elute the protein from the column in purification and analysis using column chromatography (Tsumoto, et al Biotechnology Progress. 20, 1301-1308 (2004)).

US 5 110 600 relates to a method of treating retroviral infection with L-canavanine (an analog of arginine).

It is desirable that embodiments of the present invention provide a method for conveniently producing a protein formulation with the lipid-enveloped viruses being inactivated, without impairing the quality of the obtained protein formulation.

Embodiments of the present invention further desirably provide a method for conveniently inactivating the lipid-enveloped viruses present in a protein formulation, without impairing the quality of the protein formulation.

Some embodiments of the present invention desirably provide a method for conveniently inactivating lipid-enveloped viruses present on the surface portions of an article or material.

Aspects of the invention involve bringing a protein formulation into contact with a specifically pH adjusted arginine solution at specific concentrations.

Namely, the present invention relates to a method for producing a protein formulation with the viruses being inactivated, comprising the step of exposing the protein formulation contaminated with the viruses to an aqueous solution of arginine, an arginine derivatives or a mixture thereof in a concentration ranging from 0.1 to 2 M, the aqueous solution being adjusted to pH 3.5 to 5.

Also, the present invention relates to a method for inactivating viruses present in a protein formulation, comprising the step of exposing the protein formulation contaminated with the viruses to an aqueous solution of arginine, an arginine derivatives, or a mixtures thereof in a concentration ranging from 0.1 to 2 M, the aqueous solution being adjusted to pH 3.5 to 5.

In addition, the present invention relates to a virus inactivation method comprising the step of bringing a virus-containing object into contact with an aqueous solution of arginine, an arginine derivative, or a mixture thereof in a concentration ranging from 0.1 to 2 M, the aqueous solution being adjusted to pH 3.5 to 5.

According to the present invention, it becomes possible to produce protein formulations where lipid-enveloped viruses are highly inactivated without providing many additional steps. Arginine or arginine derivatives according to claim 1 can be used as the pharmaceutical additives for protein formulations, so that arginine or arginine derivatives according to claim 1 may not be removed from the obtained protein formulations and the qualities of the obtained protein formulations are not impaired. Furthermore, arginine or arginine derivatives according to claim 1 can be used for purification of protein, so that purification of the protein can be carried out concurrently with inactivation of the lipid-enveloped viruses therein according to the present invention. The present invention also makes it possible to inactivate lipid-enveloped viruses present on the surface portions of the solids such as articles or materials, the human tissues and the tissues of other animals and plants; lipid-enveloped viruses present in the liquids such as pharmaceutical drugs in the form of a solution, syrup, liquid type foods such as refreshing drinks, mayonnaise; and lipid-enveloped viruses present in the gases such as air, under the moderate conditions in a short time, without having any adverse effect on the characteristics of the individual target object.

Embodiments of the invention are described in more detail below.

The protein formulations herein used are those made from organism-derived starting materials or made using cells and animal-derived materials in the manufacturing process, so that the manufacturing process of those protein formulations absolutely requires the step of virus inactivation. For example, the protein formulations for use in the present invention include antibodies obtained from human plasma, humanized antibodies and human antibodies prepared by gene-engineered cell culture technology, monoclonal antibodies of mouse and the like. To be more specific, there are protein formulations such as Muramomab (product name: Orthclone OKT3), Rituximab (product name: Ritaxan), Basiliximab (product name: Simulect), Daclizumab (product name: Zenapax), Palivizumab (product name: Synagis), Infliximab (product name: Remicade), Gemtuzumab zogamicn (product name: Mylotarg), Alemtuzumab (product name: Mabcampath), Adalimumab (product name: Humira), Omalizumab (product name: Xolair), Vevacizumab (product name: Avastin), Cetuximab (product name: Erbitux) and the like. The present invention is especially useful in the preparation of the humanized antibodies and human antibodies, and can also be applied to the preparation of pharmaceutical formulations from human plasma-derived protein pharmaceuticals, vaccines, enzymes for therapeutic use. More specifically, there are protein formulations made from donated blood plasma fractionation (eg. albumin, blood coagulation factor VII formulations, blood coagulation factor VIII formulations), influenza vaccines, thrombolytic agents (i.e., urokinase, tissue plasminogen activator).

Arginine and the arginine derivatives according to claim 1 used in the present invention may be provided in the form of acid addition salts. Examples of acids capable of forming the acid addition salts include hydrochloric acid, sulfuric acid. Particularly preferred is hydrochloric acid. Arginine and the arginine derivatives according to claim 1 are preferable because they are suitable for purification of the protein formulations. The arginine derivatives include Nα-acetyl-L-arginine, Nα-butyroyl-L-arginine, Nα-pivaloyl-L- arginine, Nα-valeroyl-L-arginine and Nα-butyroyl-L-arginine is most preferable.

In the present invention, the aqueous solution of arginine, the aqueous solution of arginine derivative according to claim 1, or the mixture thereof is adjusted to have a concentration of 0.1 to 2 M, more preferably 0.15 to 2 M, and most preferably 0.2 to 1.0 M. When the concentration is 0.1 M or more, significant effect of virus inactivation can be achieved. The upper limit is set to be 2 M from the economical viewpoint. The pH value of such an arginine-containing solution at 25°C, which can be determined using glass electrodes, is in the range of pH 3.5 to 5, preferably pH 3.8 to 5, and most preferably pH 4 to 4.5. Preferably, inactivation of viruses can rapidly proceed within such a pH range. The pH value of the arginine solution may be adjusted only by the selection of arginine or arginine derivatives. Further, the pH value of the solution can be adjusted by the addition of an acid such as hydrochloric acid or the like or an alkali such as sodium hydroxide. In order to more efficiently inactivate the viruses, the pH buffering ability may be imparted to the arginine solution by the addition thereto of a dilute buffer solution such as acetate or phosphate with low concentrations, for example, in the range of 5 to 50 mM.

According to the present invention, the lipid-enveloped viruses with which the protein formulation is contaminated in the course of manufacturing process of the formulation can be inactivated by bringing the protein formulation into contact with an arginine solution, an arginine derivative according to claim 1 solution or the mixture thereof which is adjusted to have a specific pH value at the concentration specified above. With respect to the way of how to expose the lipid-enveloped virus-containing protein formulation to the arginine solution as mentioned above, the protein, after subjected to purification by chromatography may be still left under the above specified pH conditions in the manufacturing process of the protein formulation. Alternatively, the arginine may be directly added to an aqueous solution containing a protein isolated from blood plasma or a cell culture supernatant so that the obtained aqueous solution may have the arginine concentration and the pH value as specified above. In the case where the protein is exposed to the arginine via the purification step using chromatography, the protein formulation may be, for example, dissolved in a phosphate buffer of a neutral pH value or diluted with the buffer solution about ten times, and then introduced into a column such as a protein A column (e.g., "HiTrap rProtein AFF, made by Amersham Bioscience K.K.) pre-equilibrated with the same buffer solution as mentioned above. After that, the column was thoroughly washed with the same buffer solution to rinse out the impurities derived from the raw materials. Then, an aqueous solution of arginine, an arginine derivative according to claim 1 or the mixture thereof, the arginine concentration and the pH value being adjusted as previously specified in the present invention is introduced into the column. The protein formulation thus desorbed may be collected. This process can conduct the virus inactivation and also the purification of protein formulation. The operating temperature may generally be in the range of 0 to 30°C, and preferably 0 to 8°C, in consideration of inactivation of the viruses, and at the same time, prevention of the protein from freezing and denaturing at low or high temperatures. The operating time may be generally in the range from about 15 minutes to about two hours, and preferably about one hour. Within the above-mentioned operating time, the viruses lose their infectivity and are finally inactivated by the action of arginine.

Also, according to the present invention, lipid-enveloped viruses present on a target object can be inactivated effectively in a short time by bringing the object into contact with an arginine solution, an arginine derivative according to claim 1 solution or the mixture thereof which is adjusted to have a particular pH at the concentration as specified above. In this case, the aqueous solution of arginine, the aqueous solution of arginine derivative according to claim 1 or the mixture thereof is adjusted to have a concentration of 0.1 to 2 M, more preferably 0.1 to 1 M, and most preferably 0.1 to 0.3 M. Such an aqueous solution of arginine in the above specified concentration is adjusted to pH 3.5 to 5, preferably pH 3.6 to 4.8, and more preferably pH 3.6 to 4.5 at 25°C. When the pH value of the arginine-containing solution is lower than 3.5, the solution becomes more irritating to the tissues of animals, and the present invention is therefore not considered to be advantageous over the conventionally known methods of using citric acid. The pH of more than 5 is unfavorable because the virus inactivating effect is decreasing.

The target object herein used covers, for example, solids, liquids, and gases, so long as the object may have the potential for being contaminated with lipid-enveloped viruses. Examples of the solid object include articles such as medical appliances, straps in trains, furniture, household electric appliances, bedding such as futon, clothes, pet animals such as dogs, cats; tissues of animals, for example, the upper or lower part of respiratory tract, oral cavity and skin of human; and tissues of plants, for example, the epidermis. Examples of the liquid object include pharmaceutical preparations such as solutions, syrups, liquid type foods such as refreshing drinks, and semi-solid type foods such as mayonnaise. The gaseous object includes air.

To bring the object contaminated with lipid-enveloped viruses into contact with an arginine-containing solution, an arginine solution, an arginine derivative according to claim 1 solution or the mixture thereof adjusted to have a particular pH at the concentration as specified above may be sprayed to the object using an atomizer, or coated to the object using a brush. Alternatively, an arginine-impregnated nonwoven fabric may be applied onto a target portion of the object or inserted into the inner side of a mask. When the arginine-containing solution is brought into contact with an object in the form of a liquid, the arginine solution may be mixed or stirred with the liquid (object) if necessary. When the liquid (object) is an oleaginous substance such as an oil or fat, an emulsifier or the like may be added so that the liquid (object) and the aqueous solution may sufficiently come in contact with each other. Also, a polymer such as methyl cellulose or the like may be added to the above-mentioned arginine aqueous solution to impart the thickening effect to the aqueous solution. This can prolong the contact time of the liquid (object) with the arginine aqueous solution, thereby ensuring a sufficient time to inactivate the viruses in the liquid (object).

The surface temperature of the tissues of human and animals is maintained at a temperature ranging from room temperature to the body temperature. When the arginine-containing solution is applied to the tissues of animals, such as the human tissues and the like, the inactivation reaction of virus, e.g., influenza virus proceeds even more rapidly than the case where the viruses are exposed to the inactivating solution at low temperatures. In this case, the inactivation reaction can be completed in about two minutes.

For example, when the influenza virus is attached to the upper respiratory tract of the human, the virus can be inactivated by nasal spray of the above-mentioned arginine solution. Although the exposure time of the tissue surfaces of the upper respiratory tract to the arginine solution is short, the virus inactivation can be achieved.

When the target object is brought into contact with the above-mentioned arginine solution, the amount of the arginine solution varies depending on the contact manner. For example, when the above-mentioned aqueous solution of arginine is nasally sprayed on the human mucosa of upper respiratory tract, the dosage of the aqueous solution is generally about 0.1 ml. The mucosa of the upper respiratory tract is exposed under the atmosphere of aqueous arginine solution for several minutes after completion of the spraying operation, so that the influenza viruses can be inactivated reliably even if they remain on the mucosa. Similarly, when the influenza viruses are attached to the exposed tissues such as the palm, those viruses can be inactivated instantaneously by spraying of about 0.1 ml of the above-mentioned aqueous arginine solution.

The amount of the aqueous solution to be sprayed may be properly determined depending upon the surface area of the target tissue. Also, when the influenza viruses are attached to the surface of appliances for example, the viruses can be inactivated instantaneously by spraying the above-mentioned aqueous solution on the surface of the target appliance at room temperature or more, in a proper amount depending upon the surface area of the target object.

The present invention is particularly effective in inactivating the lipid-enveloped viruses. For example, influenza virus, and coronavirus can be listed.

To evaluate the inactivating efficiency, a concentrated virus stock of which the viral titer is identified is first added to a protein solution. After completion of the exposure to the inactivating solution, the residual virus titer is determined by any suitable methods such as TCID₅₀ method, plaque assay method. Thus, the log reduction value (LRV), which is defined as the common logarithm (log₁₀) of the ratio of the virus load in a sample before inactivation to the virus content in the sample after inactivation is calculated, and the calculated LRV can evaluate the inactivating efficiency. The inactivating efficiency is considered to be significant when the LRV is 1 or more (ICH Harmonized Tripartite Guideline: Viral Safety Evaluation of Biotechnology Products Derived from Cell Lines of Human or Animal Origin).

When the protein formulations obtained by the method of the present invention are analyzed by gel filtration chromatography, the proteins are eluted with the same peaks appearing at the same retention time as with the case of proteins in the native state. This demonstrates that the protein formulations have not been subjected to changes in high-order structure, and association or aggregation.

By using the protein formulations obtained by the method of the present invention, it becomes possible to produce therapeutic agents, reagents for clinical laboratory test, and laboratory-use reagents for various diseases such as cancer, immune system disorders, lifestyle-related diseases and the like. Those pharmaceutical compositions may further comprise appropriate excipients, carriers, in addition to the purified antibodies obtained by the method of the present invention.

Furthermore, the present invention is also applicable to the method for producing inactivating agents capable of inactivating the viruses such as influenza virus, coronavirus, and inhibitors for viral infectious diseases related to the above-mentioned viruses. Those inactivating agents and inhibitors may also comprise pharmaceutically acceptable excipients, carriers, and may be prepared in the liquid form or the like by the conventional methods.

### Example 1

An aliquot (15 ml) of a solution prepared by dissolving purified anti-von Willebrand Factor monoclonal antibody (mouse monoclonal antibody, subclass IgG₁; WO96/17078) in Dulbecco's isotonic phosphate buffer solution (free from Ca and Mg) was subjected to overnight dialysis against 5000 ml of 5 mM sodium phosphate (pH 4.4). The above-mentioned dialysis operation was conducted twice, respectively. The dialyzed antibody fluids (inner part) were combined to yield about 30 ml. The solution thus obtained was then adjusted to have an antibody concentration of 10 mg/ml using the outer part of the dialysis. The concentration-adjusted solution (5 ml) was diluted twice with each of the buffer solutions for virus inactivation which were separately prepared, and thereafter finely adjusted to have a predetermined pH value using 2M NaOH or 2M aqueous solution of hydrochloric acid. Each of the obtained solutions was subjected to sterile filtration using a disposable filter (0.22 µm) and stored at 5°C before use. Table 1 shows the inactivating conditions of each buffer solution.

Using the Vero cell, the herpes simplex viruses type 1, strain F (HSV-1) were grown in an Eagle's minimum essential medium (MEM) containing 0.5% fetal calf serum, thereby preparing a concentrated virus suspension. The thus obtained virus-containing suspension was stored at -80°C before use. The viral titer was determined using the Vero cell in accordance with the plaque assay known from the previous report (Koyama, et. al. Virus Res. 13, 271-282 (1989)). Under ice cooling conditions, 0.95 ml of each of the virus-inactivating buffer solutions shown in Table 1 was put into a 1.5-ml plastic tube, where 0.05 ml of the concentrated HSV-1 suspension (with a virus concentration of about 10⁹ plaque forming units (PFU)/ml) was further added. After the mixture was instantaneously stirred, the mixture was kept under the ice cooling conditions for one hour. After that, the mixture was diluted 100 times with Dulbecco's isotonic phosphate buffer solution (free from Ca and Mg) containing 1% fetal calf serum to conduct pH neutralization titration, thereby terminating the virus inactivating reaction. The reaction solution was appropriately diluted with Dulbecco's isotonic phosphate buffer solution (free from Ca and Mg) containing 1% fetal calf serum and the residual HSV-1 titer (the concentration of the viruses still remaining infectious) was determined using the plaque assay previously mentioned. In accordance with the ICH Harmonized Tripartite Guideline mentioned above, the titer of HSV-1 loaded in a sample was determined after the sample had been holded for one hour under the ice cooling conditions in a Dulbecco's isotonic phosphate buffer solution instead of the virus inactivating buffer solution. The titer of HSV-1 thus obtained after completion of the holding time was regarded as a virus load in the sample before inactivation. The virus inactivating efficiency was defined as the log₁₀ of the ratio of the virus load before inactivation to that after inactivation (Table 1).

As shown in Table 1, the inactivation did not take place when the 0.1 M citrate buffer solution of pH 4.3 was employed. Even when the citrate buffer solution adjusted to pH 4.0 was employed, the degree of inactivation was just considerably slight (LRV = 1.5). The inactivation effect was sharply increased (LRV > 5.7) at pH 3.5. Those results are in good agreement with the findings from the prior art. Namely, to inactivate the viruses by use of sodium citrate, the acidic conditions corresponding to pH <4 are necessary. On the other hand, any of the buffer solutions of 1M arginine hydrochloride (pH 4.3), 0.7 M arginine hydrochloride (pH 4.0), and 0.7 M Nα-butyroyl-L-arginine (pH 4.0) showed the same level in the inactivation effect as with the sodium citrate of pH 3.5.

As mentioned above, arginine and acyl arginine are found to have a strong inactivation effect on the HSV-1 under moderately acidic conditions of around pH 4.

**Table 1**

| Inactivating Conditions | | Virus Inactivating Efficiency (LRV) |
|---|---|---|
| Buffer solution composition | pH | |
| Dulbecco's isotonic phosphate buffer solution | 7.2 | -- |
| 0.1M sodium citrate | 4.3 | 0.1 |
| 0.1M sodium citrate | 4.0 | 1.5 |
| 0.1M sodium citrate | 3.5 | >5.7 |
| 1M arginine hydrochloride | 4.3 | >5.7 |
| 0.7M arginine hydrochloride, 20mM sodium acetate | 4.0 | >5.7 |
| 0.7M Nα-butyroyl-L-arginine | 4.0 | >5.7 |

| | | |
|---|---|---|
| LRV = log₁₀ (virus load/residual virus content) Virus load: concentration of viruses in the sample retained in Dulbecco's isotonic phosphate buffer solution. Residual virus content: concentration of viruses still remaining in the sample subjected to inactivation. | | |

### Example 2

Using the MDCK cell, the Influenza A viruses/Aichi were grown in an Eagle's minimum essential medium (MEM) containing 0.1% bovine serum albumin and 4 µg/ml acetylated trypsin, thereby preparing a concentrated virus suspension. The thus obtained virus suspension was stored at -80°C before use. The viral titer was determined using the MDCK cell in accordance with the plaque assay known from the previous report (Kurokawa et. al.: Intern. J. Mol. Med. 3, 527-530 (1999)). Under ice cooling conditions, 0.95 ml of each of the same virus-inactivating buffer solutions as employed in Example 1 was put into a 1.5-ml plastic tube, where 0.05 ml of the concentrated influenza A virus suspension (with a virus titer of about 10⁸ PFU/ml) was further added. After the mixture was instantaneously stirred, the mixture was kept under the ice cooling conditions for one hour. After that, the mixture was diluted 100 times with Dulbecco's isotonic phosphate buffer solution (free from Ca and Mg) containing 0.1% bovine serum albumin to conduct pH neutralization titration, thereby terminating the virus inactivating reaction. The reaction solution was appropriately diluted with Dulbecco's isotonic phosphate buffer solution (free from Ca and Mg) containing 0.1% bovine serum albumin and the residual influenza A virus titer (the titer of the viruses still remaining infectious) was determined using the plaque assay previously mentioned. In accordance with the ICH Harmonized Tripartite Guideline mentioned above, the titer of influenza A virus loaded in a sample was determined after the sample had been retained for one hour under the ice cooling conditions in a Dulbecco's isotonic phosphate buffer solution (free from Ca and Mg) instead of the virus inactivating buffer solution. The titer of influenza A virus thus obtained after completion of the retention time was regarded as a virus load in the sample before inactivation. The virus inactivating efficiency was defined as the log₁₀ of the ratio of the virus load before inactivation to that after inactivation (Table 2).

As shown in Table 2, the virus inactivation was done by a 0.1 M citrate buffer solution at pH 4.3 although the inactivating level was slight (LRV = 1.3). This attests to the fact that the surface antigen Hemagglutinin (HA) is unstable under the acidic conditions. However, any change in the inactivating efficiency was hardly observed (LRV = 1.5) at a more acidic value of pH 4.0; and the inactivation level was still slight (LRV = 2.1) even though the citrate buffer solution was adjusted to pH 3.5. On the other hand, the inactivating effect (LRV = 2.4) obtained by the buffer solution of 1M arginine hydrochloride of pH 4.3 was found to be higher than that of the sodium citrate of pH 3.5. Furthermore, 0.7 M arginine hydrochloride (pH 4.0) and 0.7 M Nα-butyroyl-L-arginine (pH 4.0) showed even more inactivating power to achieve the LRV of 3.7.

As mentioned above, arginine and acyl arginine are also found to have strong effects in inactivating the influenza A virus under moderately acidic conditions of around pH 4.

**Table 2**

| Inactivating Conditions | | Virus Inactivating Efficiency (LRV) |
|---|---|---|
| Buffer solution composition | pH | |
| Dulbecco's isotonic phosphate buffer solution | 7.2 | -- |
| 0.1M sodium citrate | 4.3 | 1.3 |
| 0.1M sodium citrate | 4.0 | 1.5 |
| 0.1M sodium citrate | 3.5 | 2.1 |
| 1M arginine hydrochloride | 4.3 | 2.4 |
| 0.7M arginine hydrochloride, 20mM sodium acetate | 4.0 | 3.7 |
| 0.7M Nα-butyroyl-L-arginine | 4.0 | 3.7 |

| | | |
|---|---|---|
| LRV = log₁₀ (virus load/residual virus content) Virus load: concentration of viruses in the sample retained in Dulbecco's isotonic phosphate buffer solution. Residual virus content: concentration of viruses still remaining in the sample subjected to inactivation. | | |

### Example 3

Using the HSV-1 suspension prepared in the same manner as in Example 1, the relationship between the virus inactivating effect and the salt concentration was investigated. The viral titer was determined in accordance with the plaque assay in the same manner as in Example 1. As shown in Table 3, the buffer solutions of 1 M NaCl (pH 4.3) and 0.7 M NaCl (pH 4.0) were found to have extremely weak inactivating effects (LRV = 0.8 at most) unlike the buffer solutions of arginine and Nα-butyroyl-L-arginine, although the respective concentrations of those NaCl buffer solutions were the same as those of the arginine hydrochloride and Nα-butyroyl-L-arginine which exhibited sufficient inactivating effects in Examples 1 and 2. On the other hand, when the concentration of the arginine hydrochloride (pH 4.0) was changed to 0.35 M, the inactivating effect became slightly less, but the level was still sufficient (LRV = 4.2). The buffer solution of 0.35 M Nα-butyroyl-L-arginine (pH 4.0) showed an extremely strong inactivating effect (LRV > 5.5).

The above-mentioned findings demonstrate that the inactivating effects of arginine and acyl arginine are not just ascribed to the salt concentration, but based on the properties peculiar to arginine. When the buffer solutions of 0.1 M arginine hydrochloride and 0.7 M arginine hydrochloride were adjusted to pH 3.5, their respective inactivating effects exhibited the same levels as that of the 0.1 M sodium citrate (pH 3.5) as expected.

**Table 3**

| Inactivating Conditions | | Virus Inactivating Efficiency (LRV) |
|---|---|---|
| Buffer solution composition | pH | |
| Dulbecco's isotonic phosphate buffer solution | 7.2 | -- |
| 1M NaCl, 0.02M sodium acetate | 4.3 | 0 |
| 0.7M NaCl, 0.02M sodium acetate | 4.0 | 0.8 |
| 0.35M arginine hydrochloride | 4.0 | 4.2 |
| 0.35M Nα-butyroyl-L-arginine | 4.0 | >5.5 |
| 0.7M arginine hydrochloride | 3.5 | >5.5 |
| 0.1M arginine hydrochloride | 3.5 | >5.5 |

| | | |
|---|---|---|
| LRV = log₁₀ (virus load/residual virus content) Virus load: concentration of viruses in the sample retained in Dulbecco's isotonic phosphate buffer solution. Residual virus content: concentration of viruses still remaining in the sample subjected to inactivation. | | |

### Example 4

Using the concentrated HSV-1 suspension prepared in the same manner as in Example 1, the relationship between the virus inactivating effect and the concentrations of arginine or acyl arginine was investigated (Table 4). The viral titer was determined in accordance with the plaque assay in the same manner as in Example 1. As shown in Table 4, when the concentration was 0.14 M or more, both the arginine hydrochloride and the Nα-butyroyl-L-arginine showed significant inactivating effects (LRV > 1.0). When the arginine hydrochloride was compared with the Nα-butyroyl-L-arginine, for example, at a concentration of 0.28 M, the inactivating effect of the latter appeared to be significantly stronger than that of the former.

**Table 4**

| Concentration (M) | Virus Inactivating Efficiency (LRV) | |
|---|---|---|
| | Arginine hydrochloride (pH 4.0) | Nα-butyroyl-L-arginine (pH 4.0) |
| 0 | -- | -- |
| 0.07 | 0.5 | 0.7 |
| 0.14 | 1.1 | 2.2 |
| 0.21 | 2.2 | 4.3 |
| 0.28 | 3.1 | 6.0 |
| 0.35 | 4.5 | not determined |

| | | |
|---|---|---|
| LRV = log₁₀ (virus load/residual virus content) Virus load: concentration of viruses in the sample retained in Dulbecco's isotonic phosphate buffer solution. Residual virus content: concentration of viruses still remaining in the sample subjected to inactivation. | | |

### Example 5

Sendai viruses were cultured using embryonated egg, and then a virus-containing suspension was prepared by appropriately diluting with Dulbecco's isotonic phosphate buffer solution (free from Ca and Mg) containing 0.1% bovine serum albumin. The thus obtained virus suspension was stored at -80°C before use. The viral titer was determined by the plaque assay in the same manner as in Example 2 except that the MDCK cell was replaced by the Vero cell. Under ice cooling conditions, 0.95 ml of each of the virus-inactivating buffer solutions, i.e., 0.1M sodium citrate (pH 3.5), 0.7M NaCl (pH 4.0) and 0.7M Nα-butyroyl-L-arginine (pH 4.0) was put into a 1.5-ml plastic tube, where 0.05 ml of the concentrated Sendai virus suspension (with a virus titer of about 10⁸ to 10⁹ PFU/ml) was further added. The mixture was instantaneously stirred, and then kept under the ice cooling conditions for one hour. As control samples for reference, an isotonic phosphate buffer solution (pH 7.2) and a strongly acidic isotonic citrate buffer solution (pH 3.0) known to have an extremely powerful inactivating effect were used. After completion of the retention time, the reaction mixture was diluted 100 times with Dulbecco's isotonic phosphate buffer solution (free from Ca and Mg) containing 0.1% bovine serum albumin to conduct pH neutralization titration, thereby terminating the virus inactivating reaction. The reaction solution was appropriately diluted with Dulbecco's isotonic phosphate buffer solution (free from Ca and Mg) containing 0.1% bovine serum albumin and the residual Sendai virus titer (the titer of the viruses still remaining infectious) was determined using the plaque assay previously mentioned. The titer of the Sendai virus loaded in a sample was determined after the sample had been retained for one hour in a Dulbecco's isotonic phosphate buffer solution (free from Ca and Mg) instead of the virus inactivating buffer solution. This was regarded as a virus load in the sample before inactivation. The virus inactivating efficiency was defined as the log₁₀ of the ratio of the virus load before inactivation to that after inactivation (Table 5).

As is apparent from Table 5, both the buffer solutions of 0.1M sodium citrate (pH 3.5) and 0.7M NaCl (pH 4.0) had no inactivating effect, while the buffer solution of 0.7M Nα-butyroyl-L-arginine (pH 4.0) exhibited the inactivating effect (LRV > 3.7) higher than that of the isotonic citrate buffer solution (40 mM sodium citrate, 5.0 mM KCI, 125 mM NaCl, Ph 3.0; Microbiol. Immunol., 31, 123-130 (1987)) which is a strongly acidic solution capable of creating powerful inactivating conditions.

In addition to the results of Example 4, the followings demonstrate that the arginine derivative has considerably powerful effects in inactivating the viruses.

**Table 5**

| Inactivating Conditions | | Virus Inactivating Efficiency (LRV) |
|---|---|---|
| Buffer solution composition | pH | |
| Dulbecco's isotonic phosphate buffer solution | 7.2 | -- |
| Isotonic citrate buffer solution (*) | 3.0 | 3.4 |
| 0.1M sodium citrate | 3.5 | 0.3 |
| 0.7M NaCl, 0.02M sodium acetate | 4.0 | 0.1 |
| 0.7M Nα-butyroyl-L-arginine | 4.0 | >3.7 |

| | | |
|---|---|---|
| LRV = log₁₀ (virus load/residual virus content) Virus load: concentration of viruses in the sample retained in Dulbecco's isotonic phosphate buffer solution. Residual virus content: concentration of viruses still remaining in the sample subjected to inactivation. Isotonic citrate buffer solution (*): 40 mM sodium citrate, 5.0 mM KCI, 125 mM NaCl, pH 3.0 | | |

### Example 6

Using the influenza virus A/Aichi (H3N2) suspension prepared in the same manner as in Example 2, the relationship of the virus inactivating effect to the composition of the inactivating buffer solution and the pH thereof was investigated. The concentration of each buffer solution was set to 0.15M, the inactivating temperature was raised to a room temperature of 21.6°C, and the inactivating time was shortened to two minutes. A 2.2-ml plastic tube equipped with a screw cap was charged with 190 µl of each of the inactivating buffer solutions shown in Table 6 and stored under ice cooling conditions. To each of the buffer solutions, which had been separately prepared by the addition thereto of bovine serum albumin serving as a carrier protein in a concentration of 5 mg/ml, 10 µl of the influenza virus A suspension (with a virus concentration of about 10⁸ PFU/ml) was further added. After the mixture was instantaneously stirred, the mixture was placed in a thermostatic chamber of 21.6°C for two minutes. Two minutes later, each sample was immediately cooled using ice water, and subsequently diluted 100 times with Dulbecco's isotonic phosphate buffer solution (free from Ca and Mg) containing 0.1% bovine serum albumin to conduct pH neutralization titration, thereby terminating the virus inactivating reaction. The residual virus titer was determined using the plaque assay in the same manner as in Example 2. The titer of the influenza virus A loaded in a sample was determined after the sample had been retained in a Dulbecco's isotonic phosphate buffer solution (free from Ca and Mg) instead of the virus inactivating buffer solution at 21.6°C for two minutes. The thus obtained titer of the influenza virus A was regarded as a virus load in the sample before inactivation. The virus inactivating efficiency was defined as the log₁₀ of the ratio of the virus load in the sample before inactivation to that after inactivation (Table 6).

As shown in Table 6, when the pH value was set to 3.8, any buffer solutions of 0.15M PCA (pyrrolidone carboxylic acid), 0.15M arginine hydrochloride and 0.15M Nα-butyroyl-L-arginine showed high LRV values of more than 4.35 and they were found to have powerful inactivating effects. In the case of the 0.15M sodium citrate, the maximum LRV was 2.95 at most within the pH range of 3.8 to 4.2. Namely, the 0.15M sodium citrate appeared to less function than other three kinds of buffer solutions in the virus inactivation. When the pH value was raised to 4.2, the arginine hydrochloride and the Nα-butyroyl-L-arginine still maintained high LRV values of more than 3.0, although the inactivating effects were somewhat lowered in any of PCA, arginine hydrochloride and Nα-butyroyl-L-arginine.

As previously described, it has been confirmed that the influenza virus can be inactivated effectively by exposing the viruses to arginine hydrochloride or Nα-butyroyl-L-arginine at room temperature even for a short period of time, e.g., about two minutes. Those buffer solutions exhibited superior inactivating effects when compared with the sodium citrate and PCA at the same concentration.

**Table 6**

| Inactivating Conditions | | Virus Inactivating Efficiency (LRV) |
|---|---|---|
| Buffer solution composition | pH | |
| Dulbecco's isotonic phosphate buffer solution | 7.2 | -- |
| 0.15M sodium citrate | 3.8 | 2.74 |
| 0.15M sodium citrate | 4.0 | 2.66 |
| 0.15M sodium citrate | 4.2 | 2.95 |
| 0.15M PCA | 3.8 | > 4.35 |
| 0.15M PCA | 4.0 | 4.05 |
| 0.15M PCA | 4.2 | 2.95 |
| 0.15M arginine hydrochloride | 3.8 | > 4.35 |
| 0.15M arginine hydrochloride | 4.0 | 3.87 |
| 0.15M arginine hydrochloride | 4.2 | 3.24 |
| 0.15M Nα-butyroyl-L-arginine | 3.8 | > 4.35 |
| 0.15M Nα-butyroyl-L-arginine | 4.0 | 4.05 |
| 0.15M Nα-butyroyl-L-arginine | 4.2 | 3.57 |

| | | |
|---|---|---|
| LRV = log₁₀ (virus load/residual virus content) Virus load: concentration of viruses in the sample retained in Dulbecco's isotonic phosphate buffer solution. Residual virus content: concentration of viruses still remaining in the sample subjected to inactivation. | | |

## Claims

1. A method comprising exposing a protein formulation contaminated with lipid-enveloped viruses to a 0.1 to 2M aqueous solution of arginine, an acylated arginine selected from the group consisting of Nα-acetyl-L-arginine, Nα-butyroyl-L-arginine, Nα-pivaloyl-L-arginine, Nα-valeroyl-L-arginine and Nα-caproyl-L-arginine, or a mixture thereof, the aqueous solution being adjusted to pH 3.5 to 5, thereby to produce a protein formulation with viruses being inactivated.

2. The method of claim 1, wherein the aqueous solution is adjusted to pH 4 to 4.5.

3. The method of claim 1 or claim 2, wherein the concentration of the arginine or the acylated arginine in the aqueous solution of arginine, acylated arginine or mixture thereof is 0.15 to 2 M.

4. The method of any one of the preceding claims, wherein the protein formulation is a humanized antibody or a human antibody.

5. A method of treating an object contaminated with lipid-enveloped viruses to inactivate the virus, comprising bringing the object into contact with a 0.1 to 2M aqueous solution of arginine, an acylated arginine, selected from the group consisting of Nα-acetyl-L-arginine, Nα-butyroyl-L-arginine, Nα-pivaloyl-L-arginine, Nα-valeroyl-L-arginine and Nα-caproyl-L-arginine, or of a mixture thereof, the aqueous solution being adjusted to pH 3.5 to 5, provided that the method is not one of treating the human or animal body by therapy.

6. The method of claim 5, wherein the object is a solid, liquid or gas.

7. The method of claim 6, wherein the solid is an article or a tissue of animals or plants.

8. The method of any one of claims 5 to 7, wherein the aqueous solution is adjusted to pH 3.6 to 4.5.

9. The method of any one of claims 5 to 8, wherein the concentration of the arginine or the acylated arginine in the aqueous solution of arginine, acylated arginine or mixture thereof is 0.15 to 2M.

10. Use of arginine, an acylated arginine, selected from the group consisting of Nα-acetyl-L-arginine, Nα-butyroyl-L-arginine, Nα-pivaloyl-L-arginine, Nα-valeroyl-L-arginine and Nα-caproyl-L-arginine, or of a mixture thereof, in the manufacture of a 0.1 to 2M aqueous solution of pH 3.5 to 5 for inactivation of lipid-enveloped viruses in the human or animal body.

## Patentansprüche

1. Verfahren, welches umfasst, dass eine mit Lipid-umhüllten Viren kontaminierte Proteinformulierung einer 0,1 bis 2 M wässrigen Lösung von Arginin, einem acylierten Arginin, ausgewählt aus der Gruppe, die aus Nα-Acetyl-L-arginin, Nα-Butyroyl-L-arginin, Nα-Pivaloyl-L-arginin, Nα-Valeroyl-L-arginin und Nα-Caproyl-L-arginin besteht, oder einem Gemisch davon ausgesetzt wird, wobei die wässrige Lösung auf pH 3,5 bis 5 eingestellt wird, wodurch eine Proteinformulierung mit inaktivierten Viren hergestellt wird.

2. Verfahren nach Anspruch 1, wobei die wässrige Lösung auf pH 4 bis 4,5 eingestellt wird.

3. Verfahren nach Anspruch 1 oder 2, wobei die Konzentration des Arginins oder des acylierten Arginins in der wässrigen Lösung von Arginin, acyliertem Arginin oder einem Gemisch davon 0,15 bis 2 M ist.

4. Verfahren nach einem der vorstehenden Ansprüche, wobei die Proteinformulierung ein humanisierter Antikörper oder ein menschlicher Antikörper ist.

5. Verfahren zum Behandeln eines mit Lipid-umhüllten Viren kontaminierten Gegenstandes zur Inaktivierung des Virus, welches umfasst, dass der Gegenstand mit einer 0,1 bis 2 M wässrigen Lösung von Arginin, einem acylierten Arginin, ausgewählt aus der Gruppe, die aus Nα-Acetyl-L-arginin, Nα-Butyryl-L-arginin, Nα-Pivaloyl-L-arginin, Nα-Valeroyl-L-arginin und Nα-Caproyl-L-arginin besteht, oder einem Gemisch davon kontaktiert wird, wobei die wässrige Lösung auf pH 3,5 bis 5 eingestellt wird, mit der Maßgabe, dass das Verfahren nicht ein Verfahren zum Behandeln des menschlichen oder tierischen Körpers durch Therapie ist.

6. Verfahren nach Anspruch 5, wobei der Gegenstand ein Feststoff, eine Flüssigkeit oder ein Gas ist.

7. Verfahren nach Anspruch 6, wobei der Feststoff ein Gegenstand oder ein Gewebe von Tieren oder Pflanzen ist.

8. Verfahren nach einem der Ansprüche 5 bis 7, wobei die wässrige Lösung auf pH 3,6 bis 4,5 eingestellt wird.

9. Verfahren nach einem der Ansprüche 5 bis 8, wobei die Konzentration des Arginins oder des acylierten Arginins in der wässrigen Lösung von Arginin, acyliertem Arginin oder einem Gemisch davon 0,15 bis 2 M ist.

10. Verwendung von Arginin, einem acylierten Arginin, ausgewählt aus der Gruppe, die aus Nα-Acetyl-L-Arginin, Nα-Butyroyl-L-arginin, Nα-Pivaloyl-L-arginin, Nα-Valeroyl-L-arginin und Nα-Caproyl-L-arginin besteht, oder einem Gemisch davon in der Herstellung einer 0,1 bis 2 M wässrigen Lösung von pH 3,5 bis 5 zur Inaktivierung Lipid-umhüllter Viren im menschlichen oder tierischen Körper.

## Revendications

1. Procédé comprenant l'exposition d'une formulation de protéine contaminée avec des virus à enveloppe lipidique à une solution aqueuse 0,1 à 2 M d'arginine, d'une arginine acylée choisie dans le groupe consistant en Nα-acétyl-L-arginine, Nα-butyroyl-L-arginine, Nα-pivaloyl-L-arginine, Nα-valéroyl-L-arginine et Nα-caproyl-L-arginine, ou d'un mélange de celles-ci, la solution aqueuse étant ajustée à pH 3,5 à 5, pour produire une formulation de protéine avec des virus inactivés.

2. Procédé selon la revendication 1, où la solution aqueuse est ajustée à pH 4 à 4,5.

3. Procédé selon la revendication 1 ou la revendication 2, où la concentration de l'arginine ou de l'arginine acylée dans la solution aqueuse d'arginine, d'arginine acylée ou d'un mélange de celles-ci est 0,15 à 2 M.

4. Procédé selon l'une quelconque des revendications précédentes où la formulation de protéine est un anticorps humanisé ou un anticorps humain.

5. Procédé de traitement d'un objet contaminé avec des virus à enveloppe lipidique pour inactiver le virus, comprenant la mise en contact de l'objet avec une solution aqueuse 0,1 à 2 M d'arginine, d'une arginine acylée choisie dans le groupe consistant en Nα-acétyl-L-arginine, Nα-butyroyl-L-arginine, Nα-pivaloyl-L-arginine, Nα-valéroyl-L-arginine et Nα-caproyl-L-arginine, ou d'un mélange de celles-ci, la solution aqueuse étant ajustée à pH 3,5 à 5, à condition que le procédé ne soit pas un procédé de traitement du corps humain ou animal par thérapie.

6. Procédé selon la revendication 5 où l'objet est un solide, un liquide ou un gaz.

7. Procédé selon la revendication 6 où le solide est un article ou un tissu d'animaux ou de plantes.

8. Procédé selon l'une quelconque des revendications 5 à 7, où la solution aqueuse est ajustée à pH 3,6 à 4,5.

9. Procédé selon l'une quelconque des revendications 5-8, où la concentration de l'arginine ou de l'arginine acylée dans la solution aqueuse d'arginine, d'arginine acylée ou d'un mélange de celles-ci est 0,15 à 2 M.

10. Utilisation de l'arginine, d'une arginine acylée choisie dans le groupe consistant en Nα-acétyl-L-arginine, Nα-butyroyl-L-arginine, Nα-pivaloyl-L-arginine, Nα-valéroyl-L-arginine et Nα-caproyl-L-arginine, ou d'un mélange de celles-ci, dans la fabrication d'une solution aqueuse 0,1 à 2 M de pH 3,5 à 5 pour l'inactivation de virus à enveloppe lipidique dans le corps humain ou animal.
